# EUROPEAN PATENT APPLICATION

(11) **EP 3 718 568 A1**
(43) Date of publication of application: **07.10.2020**
(21) Application number: 18884374.2
(22) Date of filing: 28.11.2018
(51) Int. Cl.: A61K 39/395, A61P 1/00, A61P 9/00, A61P 9/10, A61P 11/00, A61P 13/12, A61P 25/00, A61P 27/02, A61P 43/00, C07K 16/28, C12N 5/0786

(54) **ACTIVITY MODULATOR**

(30) Priority: 30.11.2017 JP 2017229958
(71) Applicant: University of Tsukuba, Ibaraki 305-8577 (JP)
(72) Inventor: SHIBUYA, Akira, Tsukuba-shi Ibaraki 305-8577 (JP); ODA, Chigusa, Tsukuba-shi Ibaraki 305-8577 (JP); ABE, Fumie, Tsukuba-shi Ibaraki 305-8577 (JP); FUJIYAMA, Satoshi, Tsukuba-shi Ibaraki 305-8577 (JP)
(74) Representative: Dehns
(86) International application number: PCT/JP2018/043862
(87) International publication number: WO 2019/107445

(57) **Abstract**

The present invention relates to: an activity modulator which is a means for modulating the activity of CD300b-dependent phagocytosis; and medicine for the treatment or prevention of a disease or condition in which the activity is involved. The activity modulator comprises a CD300a-binding substance and modulates CD300b-dependent phagocytosis signals in a macrophage.

## Description

### Technical Field

The present invention relates to an activity modulator comprising a CD300a-binding substance and medicine for ischemic diseases.

### Background Art

Many people suffer from ischemic diseases in Japan against the backdrop of aging, the westernization of lifestyle and the like, which occupy the top causes of death and is a burden on the medical economy. An ischemic disease is a disease caused by an oxygen/nutrient deficiency due to a rapid decrease in blood flow caused by temporary or continuous occlusion or stenosis of blood vessels in an organ such as the heart or brain, and examples of typical ischemic diseases include myocardial infarction and cerebral infarction.

Apoptosis is one of the mechanisms of cell death related to the maintenance of homeostasis in the living body, and is known to be triggered in cells exposed to a deteriorated in vivo environment, for example, oxygen/nutrient deficiency caused by the ischemic disease. Cells that have induced apoptosis are phagocytized by being recognized by phagocytes such as macrophages, and are quickly removed.

Removal of apoptotic cells by macrophages or the like by rapid phagocytosis greatly contributes to the suppression of the pathological progression of ischemic diseases. When cells have induced apoptosis, macrophages are attracted and apoptotic cells are removed from the body (clearance), which reduces the load on surrounding cells and tissues. When such clearance of apoptotic cells is not properly induced, the apoptotic cells and their contents accumulate in the surrounding area, causing a load on the surrounding cells and tissues and causing damage to the surrounding tissues. Thus, the phagocytosis of apoptotic cells by macrophages is extremely important for maintaining the homeostasis of the living body.

The cells that have induced apoptosis are known to have an exposed phosphatidylserine (PS), which is a phospholipid present inside the cell membrane in normal cells, on the cell surface. This PS is known to mediate a so-called "eat me" signal to phagocytes such as macrophages, and macrophages recognize PS on apoptotic cells, which allows them to phagocytize the apoptotic cells (Non Patent Literatures 1 and 2) .

Mer tyrosine kinase (MerTK), which belongs to the receptor tyrosine kinase family, is known as one of the factors that receive the "eat me" signal in macrophages. MerTK enhances phagocytosis by macrophages by binding to PS on the apoptotic cell surface via its ligands gas6 and protein6. For example, it was found that macrophages prepared from mice lacking MerTK cannot phagocytize apoptotic cells (Non Patent Literature 3).

It has also been reported that CD300b (CLM7/LMIR5), a member of the CD300 family, which is a receptor family protein, recognizes PS on the apoptotic cell surface as a ligand and modulates the phagocytosis of apoptotic cells (Non Patent Literature 4).

On the other hand, regarding CD300a, which is also a member of the CD300 receptor family, the present inventors have reported that CD300a binds to PS in mast cells or the like, which are immune system cells, thereby enhancing the transduction of immunosuppressive signals (Patent Literature 1), and that CD300a of macrophages does not promote phagocytosis even when bound to PS (Non Patent Literature 5).

### Citation List

### Patent Literature

Patent Literature 1: International Publication No. WO 2013/077186

### Non Patent Literature

Non Patent Literature 1: V.A.Fadok et al. J Immunol 148, 2207 Apr 1,1992
Non Patent Literature 2: J.Savill,I.Dransfield, C.Gregory, C.Haslett, Nat Rev Immunol 2, 965, 2002
Non Patent Literature 3: Nishi et al. Mol. Cell. Biol. April 2014 vol. 34 no. 8 1512-1520
Non Patent Literature 4: Murakami et al. Cell Death and Differentiation (2014) 21, 1746-1757
Non Patent Literature 5: Nakahashi-Oda et al. J. Exp. Med., 209, 1493-1503

### Summary of Invention

### Technical Problem

The present invention aims to elucidate the activation control mechanism of CD300b-dependent phagocytosis, and to provide an activity modulator which is a means for modulating the activity of the CD300b-dependent phagocytosis, and medicine for the treatment or prevention of a disease or condition in which the activity is involved.

### Solution to Problem

As a result of intensive studies to elucidate the activation control mechanism of CD300b-dependent phagocytosis in macrophage phagocytosis, the present inventors have found that CD300a activated by binding to PS suppresses CD300b-dependent phagocytosis in macrophages.

Furthermore, the present inventors considered that when ischemia occurs in the living body, CD300b-dependent phagocytosis is suppressed by CD300a, and the cells having induced apoptosis due to the ischemia and their intracellular substances accumulate in the tissues, which causes or aggravates the ischemic disease. From this, the present inventors came to the conclusion that it is possible to treat ischemic diseases by modulating the activity of CD300a using an anti-CD300a antibody and controlling CD300b-dependent phagocytosis, and that the anti-CD300a antibody can be a tool for treating ischemic diseases, which led to the present invention.

According to the present invention made based on these findings, for example, the activity modulators and medicine shown in the following [1] to [9] are provided.
[1] An activity modulator for modulating CD300b-dependent phagocytosis signals in a macrophage, comprising a CD300a-binding substance.
[2] The activity modulator according to [1], wherein the CD300a-binding substance is a substance that inhibits the activity of CD300a in macrophages.
[3] The activity modulator according to [1] or [2], wherein the CD300a-binding substance is a substance that inhibits the binding of CD300a to phosphatidylserine.
[4] The activity modulator according to any one of [1] to [3], wherein the CD300a-binding substance is an antibody or a peptide.
[5] The activity modulator according to any one of [1] to [3], wherein the CD300a-binding substance is an anti-CD300a antibody.
[6] The activity modulator according to [5], wherein the anti-CD300a antibody is a CD300a-neutralizing antibody.
[7] The activity modulator according to [5] or [6], wherein the anti-CD300a antibody is an anti-CD300a monoclonal antibody.
[8] A medicine for ischemic diseases comprising the activity modulator according to any one of [1] to [7].
[9] The medicine for ischemic diseases according to [8], which is a medicine for treating or preventing one or more selected from ischemic heart disease, ischemic brain disease, ischemic bowel disease, ischemic kidney disease, limb ischemia, retinal ischemia, ischemic lung disease and spinal cord ischemia.

### Advantageous Effects of Invention

According to the present invention, an activity modulator and medicine for ischemic diseases, which modulates the signal transduction relating to the CD300b-dependent phagocytosis of macrophages, can be provided.

### Brief Description of Drawings

[Figure 1] Figure 1 shows the results of flow cytometry analysis obtained in Reference Example 1. The results for the cell group treated with anti-MerTk antibody + control antibody are indicated as circles, and the results for the cell group treated with anti-MerTk antibody + anti-CD300a antibody are indicated as squares.
[Figure 2] Figure 2A is a graph showing the measured values of urea nitrogen (BUN) and the serum concentration of creatinine (Cre) in CD300a^{fl/fl} mice (control mice) and CD300a^{fl/fl} Lysm-Cre mice 24 hours after the reperfusion treatment after performing a renal ischemia treatment in Reference Example 2. Figure 2B is a graph showing the percent survival after renal ischemia treatment/reperfusion treatment for each mouse.
[Figure 3] Figure 3 shows the stained images of hematoxylin-eosin staining in kidney tissue sections of CD300a^{fl/fl} mice (control mice) and CD300a^{fl/fl} Lysm-Cre mice before the renal ischemia treatment (naive), and 24 hours and 72 hours after the renal ischemia treatment and reperfusion treatment, and the stained images of TUNEL staining 24 hours after the ischemia treatment and reperfusion treatment, in Reference Example 3.
[Figure 4] Figure 4 is a graph showing the measured values of urea nitrogen (BUN) and the serum concentration of creatinine (Cre) 24 hours after the reperfusion treatment after performing a renal ischemia treatment in Example 1. The anti-CD300a antibody administration group is indicated by a triangle, and the non-administration group is indicated by a square.
[Figure 5] Figure 5 is a graph representing the scores of neurological findings in CD300a^{fl/fl} Lysm-Cre mice and CD300a^{fl/fl} mice (control mice) subjected to a transient cerebral ischemia treatment in Reference Example 4.
[Figure 6] Figure 6 is a graph representing the scores of neurological findings in mice subjected to a transient cerebral ischemia treatment in Example 2. The anti-CD300a antibody administration group is indicated by a square, and the non-administration group is indicated by a circle.
[Figure 7] Figure 7A is a graph representing the scores of neurological findings in mice subjected to a transient cerebral ischemia treatment in Example 3. The anti-CD300a antibody administration group is indicated by a square, and the non-administration group is indicated by a circle.
Figure 7B is a graph showing the stained images of hematoxylin-eosin staining in a cerebral tissue specimen from an anti-CD300a antibody-administered or non-administered mouse produced in Example 3, and the analysis results thereof.
[Figure 8] Figure 8 is a graph representing the survival rate of a myocardial infarction model using CD300a^{fl/fl} Lysm-Cre mice and wild type mice (C57BL/6J) produced in Reference Example 5.

### Description of Embodiments

The activity modulator and medicine for ischemic diseases of the present invention modulates CD300b-dependent phagocytosis signals of in a macrophage. Specifically, by modulating CD300b-dependent phagocytosis signal transduction, it is involved in the removal (clearance) of apoptotic cells at the ischemic site by phagocytosis.

The activity modulator and medicine for ischemic diseases of the present invention is described in detail below.

In the present specification, "ischemia" means a deficiency state of tissue oxygen, nutrients or the like due to a decrease in the amount of arterial blood supplied to the tissue due to the stenosis or occlusion of blood vessels, and "ischemic site" means a site where the amount of arterial blood is reduced by ischemia, and as a result, nutrients, oxygen, or the like are reduced.

When CD300b on the surface of a macrophage is activated by PS on cells in which apoptosis was induced by ischemia, CD300b associates with adapter molecule DAP12. As a result, the tyrosine present in the ITAM (immunoreceptor tyrosine-based activating motif) of DAP12 is phosphorylated, which allows to recruit and activate the Syk family kinase having a SH2 domain, and activating the downstream PI3K/Akt pathway allows to enhance phagocytosis by macrophages.

On the other hand, CD300a recruits the tyrosine phosphatase SHP-1 through an ITIM (Immunoreceptor tyrosine-based inhibitory motif) sequence in its intracellular domain by binding to PS and becoming activated. As a result, CD300b-dependent phagocytosis signals are suppressed by inhibiting the phosphorylation of tyrosine present in the ITAM of DAP12.

### <Activity modulator>

The "activity modulator" of the present invention comprises a CD300a-binding substance and modulates CD300b-dependent phagocytosis signals of in a macrophage. Specifically, the activity modulator of the present invention promotes CD300b-dependent phagocytosis signal transduction by binding the CD300a-binding substance contained in the activity modulator to CD300a and by inhibiting (neutralizing) the binding of CD300a to PS.

### <CD300a-binding substance>

It is preferable that the CD300a-binding substance is an substance that inhibits the activity of CD300a in macrophages, and further preferably an substance that inhibits the binding of CD300a to PS. The substance that inhibits the activity of CD300a is not particularly limited, but is preferably an antibody or a peptide, and more preferably an anti-CD300a antibody.

### <Anti-CD300a antibody>

The anti-CD300a antibody used in the activity modulator of the present invention is an antibody that specifically recognizes CD300a as an antigen. The anti-CD300a antibody may be a free antibody, or may be an antibody on which other optional agents (for example, auxiliary agents and the like) are directly or indirectly immobilized. One type of specific monoclonal antibody or a combination of two or more monoclonal antibodies (or polyclonal antibodies) may be contained in the activity modulator, but it is preferable that one type of monoclonal antibody is contained from the viewpoint of specificity.

The anti-CD300a antibody is not particularly limited as long as it is an antibody exhibiting the effect of the present invention, that is, having a function of inhibiting or suppressing the binding of CD300a to PS (neutralization function), and in particular, it is preferable to select a neutralizing antibody to CD300a. The neutralizing antibody can be prepared by known methods. When a monoclonal antibody is selected as the anti-CD300a antibody, the anti-CD300a monoclonal antibody is generally prepared by a procedure such as immunization using CD300a, preparation of hybridoma, screening, culture, and collection. From these, an appropriate monoclonal antibody having a suitable neutralization function between CD300a and PS and exhibiting the effect of the present invention should be selected.

When a polyclonal antibody is selected as the anti-CD300a antibody, a commonly used polyclonal antibody prepared by making the specific sequence portion containing the epitope of the antigen molecule as the immunizing antigen can be used as the polyclonal antibody, but is not limited thereto, and for example, a mixture of two or more monoclonal antibodies may be used as an alternative (equivalent) to a polyclonal antibody. The kind of animal (immune animal) producing the antibody is not particularly limited, but may be selected from mice, rats, guinea pigs, rabbits, goats, sheep, or the like, as conventionally.

The anti-CD300a antibody does no need to be an antibody having a full length as a natural antibody, as long as it is an antibody having the function of inhibiting or suppressing the binding of CD300a to PS (neutralization function) and exhibits the effect of the present invention, and may be an antibody fragment or derivative. That is, the term "antibody" in the present specification includes not only full-length antibodies but also derivatives of antibody fragments (Fab fragments or F(ab')₂ and the like), chimeric antibodies (humanized antibodies and the like), multifunctional antibodies and the like.

### <Peptide>

It is preferable that the peptide used as the CD300a-binding substance in the present invention is a peptide that inhibits the activity of CD300a in macrophages, and further preferably a peptide that inhibits the binding of CD300a to PS. For example, it is preferable to select a peptide that specifically binds to CD300a, and specifically, it is preferably a peptide that can be a ligand for CD300a and that inhibits the activity of CD300a in macrophages.

### <Medicine for ischemic diseases>

The "medicine for ischemic diseases" of the present invention contains the activity modulator as an active ingredient. The CD300b-dependent phagocytosis signal transduction is promoted by the activity modulator, thereby rapidly removing (clearing) apoptotic cells at the ischemic site by phagocytosis, which allows to suppress the accumulation of apoptotic cells in the tissue and to treat or prevent ischemic diseases.

"Treatment" includes curing as well as alleviating (relieving) a disease or condition, and "prevention" includes preventing a disease or condition from occurring, as well as preventing its recurrence after curing the disease or condition.

The subject to receive the medicine for ischemic diseases of the present invention may be either human or non-human (for example, mammals such as mice), but is preferably human, further preferably a human suffering from or at risk of suffering from an ischemic disease.

The "ischemic disease", which is the target disease of the medicine for ischemic diseases of the present invention, refers to a state in which the ischemia of a tissue continues due to arterial stenosis or occlusion, or to an unfavorable state caused by such ischemia, regardless of the presence or absence of subjective symptoms. Examples of the ischemic disease include, but are not particularly limited to, ischemic heart diseases such as angina pectoris and myocardial infarction, ischemic brain diseases such as cerebral infarction and moyamoya disease, ischemic kidney diseases such as renal failure, ischemic bowel diseases such as ischemic colitis or mesenteric arterial occlusion, limb ischemia such as gangrene or arteriosclerosis obliterans, retinal ischemia such as diabetic retinopathy, ischemic lung diseases, spinal cord ischemia, or the states with the signs thereof.

The administration site of the medicine for ischemic diseases of the present invention can be selected according to the disease or condition that is the target of administration, and is not particularly limited. For example, it may be administered systemically by administering in the blood, it may be administered to or near the submucosal tissue or connective tissue at the site where the ischemia is occurring, or it may be administered directly to the site where the ischemia is occurring. As the administration method, both oral administration and parenteral administration are possible, but parenteral administration is preferable, for example, intravenous injection, subcutaneous injection, local injection, intracerebroventricular administration and the like can be performed, and intravenous injection, that is, systemic administration is most preferable.

The medicine for ischemic diseases of the present invention can be administered alone or can be used in combination with other medicines such as thrombolytic agents.

The dose of the medicine for ischemic diseases of the present invention is not particularly limited, but is preferably as small as possible within the range to obtain the necessary therapeutic effect, from the economical viewpoint and the viewpoint of avoiding side effects as much as possible. Specifically, it is preferable to select it appropriately according to the degree of the disease, the patient's weight, age, and sex, and to increase or decrease it as appropriate according to the degree of improvement, etc. Moreover, the frequency of administration is preferably 1 to several times per day.

Similarly, the amount of anti-CD300a antibody contained in the medicine for ischemic diseases of the present invention can be appropriately selected. For example, it is preferably 10 to 100 mg, preferably 20 to 50 mg per kg of human or animal to be administered.

The form of the medicine for ischemic diseases of the present invention is not particularly limited, and for example, is preferably a solution or dispersion dissolved or dispersed in a diluent so as to have the desired viscosity or concentration of the contained components, and may be sterilized by filtration with a filter according to a conventional method, or may be sterilized by blending a bactericide or the like. Moreover, it may be in the form of an extemporaneous preparation, for example, it can be prepared as a solid preparation by a freeze-drying method or the like, and can be administered after being dissolved or dispersed in a diluent before use.

The "medicine for ischemic diseases" of the present invention may further contain a pharmaceutically acceptable additive as necessary. For example, it may contain a diluent, a carrier or an excipient.

The pharmaceutically acceptable additive is not particularly limited as long as it is not contrary to the object of the present invention, and examples thereof include diluents, carriers, excipients, stabilizers, preservatives, buffers, emulsifiers such as surfactants, coloring agents, thickeners, solubilizing agents such as glycols, and isotonic agents such as sodium chloride and glycerin.

Specific examples of additives include lactose, glucose, sucrose, sorbitol, mannitol, starch, gum arabic, calcium phosphate, inactive alginates, tragacanth, gelatin, calcium silicate, crystalline cellulose, polyvinylpyrrolidone, cellulose, water syrup, water, water/ethanol, water/glycol, water/polyethylene, hypertonic saline, glycol, propylene glycol, methyl cellulose, methylhydroxybenzoate, propylhydroxybenzoate, talc, magnesium stearate, fatty substances such as mineral oil or hard fats, or a suitable mixture thereof.

The forms that can be administered parenterally should be sterile and free of physiologically unacceptable agents, and should have low osmolality to minimize irritation or other side effects upon administration, and therefore the solution should preferably be isotonic, or slightly hypertonic, for example hypertonic saline (physiological saline).

### Examples

Hereafter, suitable aspects of the present invention are described more specifically based on examples, however, the present invention is not limited to these examples.

### [Reference Example 1]

Apoptosis was induced by adding 10 µM dexamethasone to thymocytes isolated from thymus samples collected from wild type mice (C57BL/6J) and culturing for 12 hours, and the thymocytes were further labeled with pHrodo (trademark) dye.

On the other hand, peritoneal exudate cells of wild type or CD300b KO mice were collected and cultured for 30 minutes to adhere macrophages to the culture dish.

1.5 x 10⁶ labeled thymocytes and 1.5 x 10⁵ macrophages were cultured for 90 minutes in the presence of 20 ng/ml of anti-MerTk neutralizing antibody (2B10C42, Biolegend) and anti-CD300a antibody (TKA139, Chugai Pharmaceutical Co., Ltd.), or in the presence of 20 ng/ml of anti-MerTk antibody (2B10C42, Biolegend) and 1 ng/ml of a control antibody (IC17-mouse IgG1 sFc, Chugai Pharmaceutical Co., Ltd.). Then, the total number of macrophages positive for anti-CD11b antibody (M1/70, TONBO bioscience) and anti-F4/80 antibody (BM8, Biolegend) and the number of pHrodo positive cells were counted using flow cytometry (BD FACSAria (trademark), BD Biosciences). Thereafter, the ratio of the latter to the former was calculated, and the value was defined as the ratio of macrophages having phagocytized apoptotic cells (FlowJo (registered trademark), FlowJo LLC).

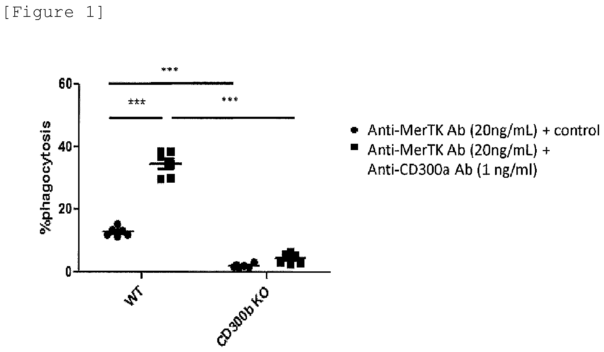

The results are shown in Figure 1.

The macrophages derived from CD300b KO mice have a significantly lower phagocytosis rate of apoptotic cells compared to WT macrophages, regardless of the presence or absence of an anti-CD300a antibody.

Here, in cells in which CD300a was neutralized with an anti-CD300a antibody, the phagocytosis rate of apoptotic cells significantly increased in WT macrophages, however, an increase in the phagocytosis rate was not observed in CD300b KO macrophages.

Therefore, it was found that macrophage phagocytosis aided by CD300b is enhanced by anti-CD300a antibody.

### [Reference Example 2]

A CD300a^{fl/fl} Lysm-Cre mouse, which is a macrophage-specific CD300a conditional knockout mouse, was produced by crossing a CD300a^{fl/fl} mouse (produced by University of Tsukuba's Laboratory Animal Resource Center) with a Lysm-Cre mouse (Jackson laboratory).

The abdominal cavity of each of the 8-14 week old CD300a^{fl/fl} mice (control mice) and CD300a^{fl/fl} Lysm-Cre mice were opened under anesthesia, and the bilateral renal artery was occluded using clips (renal ischemia treatment).

After 25 or 35 minutes of occlusion, the clips were removed to release the occlusion (reperfusion treatment) and the abdomen was closed.

Urea nitrogen (BUN) and the serum concentration of creatinine (Cre) 24 hours after the reperfusion treatment were measured for each mouse.

In addition, for the mice that were occluded for 35 minutes and underwent reperfusion treatment, the survival rate after the treatment was also followed up.

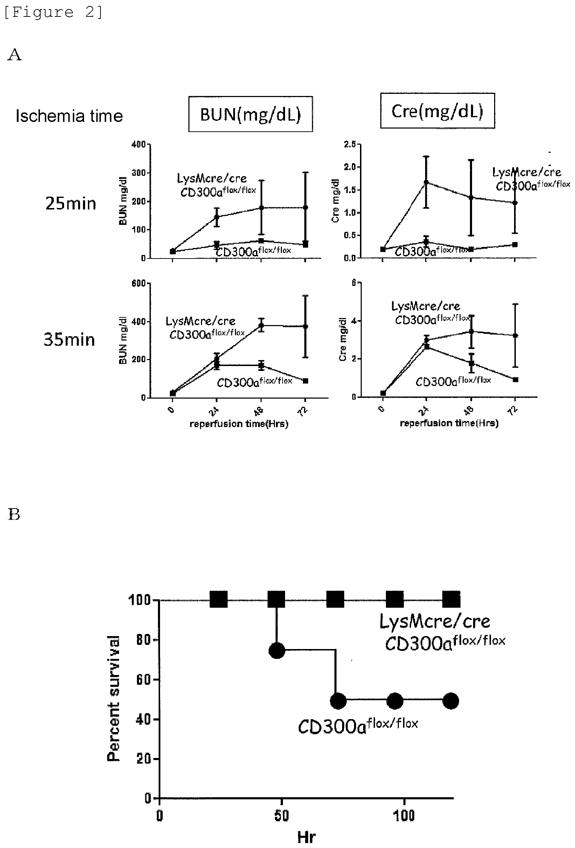

The results are shown in Figure 2.

These results shows that acute kidney injury after renal ischemia treatment is suppressed and the survival rate is maintained in mice in which CD300a is deficient in macrophages, compared to the controls.

### [Reference Example 3]

The bilateral renal arteries of each of the CD300a^{fl/fl} Lysm-Cre mice and CD300a^{fl/fl} mice (control mice) produced by the same method as in Reference Example 2 were occluded for 30 minutes, and then treated for reperfusion by the same method as in Reference Example 2. The kidneys were collected 24 hours and 72 hours after the reperfusion treatment, and tissue specimens were prepared according to a conventional method, followed by hematoxylin-eosin staining or TUNEL staining (Apoptag, Millipore).

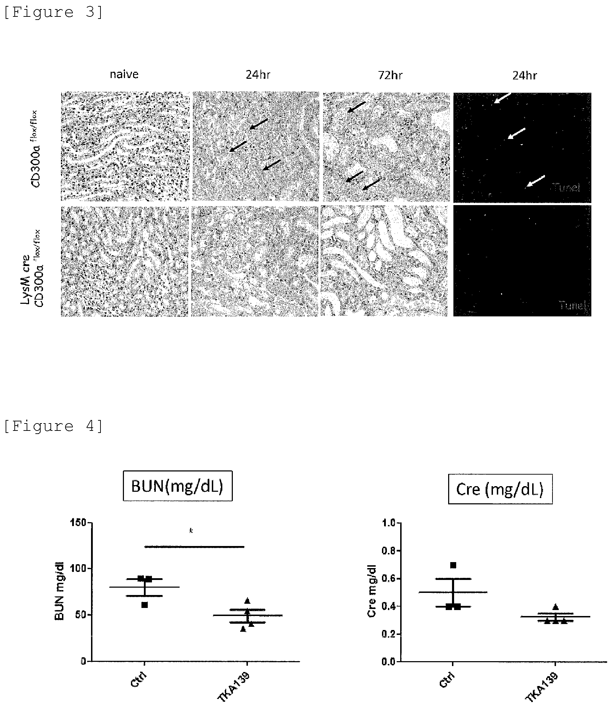

The results are shown in Figure 3. It was found that apoptosis in the tissue after renal ischemia treatment was suppressed and the accumulation of apoptotic cells in the tissue was also suppressed in mice in which CD300a is deficient in macrophages, compared to the controls.

### [Example 1]

A control antibody (IC17-mouse IgG1 sFc, Chugai Pharmaceutical Co., Ltd.) or an anti-CD300a antibody (TKA139, Chugai Pharmaceutical Co., Ltd.) was intravenously administered at 20 mg/kg to wild type mice (C57BL/6J). 3 hours after administration, a renal ischemia treatment followed by a reperfusion treatment 30 minutes later were performed with the same method as in Reference Example 2, and urea nitrogen (BUN) and the serum concentrations of creatinine (Cre) after 24 hours were each measured.

The results are shown in Figure 4. It was found that acute kidney injury after renal ischemia treatment can be suppressed by administering anti-CD300a antibody to mice in advance.

### [Reference Example 4]

For each of the CD300a^{fl/fl} Lysm-Cre mice and CD300a^{fl/fl} mice (control mice) produced by the same method as in Reference Example 2, a right middle cerebral artery occlusion (MCAO) model was produced by opening the neck under anesthesia, and placing a silicon-coated monofilament catheter (Doccol Corporation) by inserting it from the carotid artery to occlude the right middle cerebral artery (transient cerebral ischemia treatment) (Shichita et al. Nat Med 2016). 60 minutes after the occlusion treatment, the catheter was removed to reperfuse the cerebral blood flow, and the surgical wound was closed.

Thereafter, the mice were observed over the days, and the neurological findings were evaluated based on the following neurological scores with reference to Bederson et al. Stroke 1986.

0: No clear symptoms, 1: Forelimb flexion, 2: Decreased resistance to lateral push without circling, 3: Circling, 4: Forced circling, 5: Almost no movement, 6: Dead.

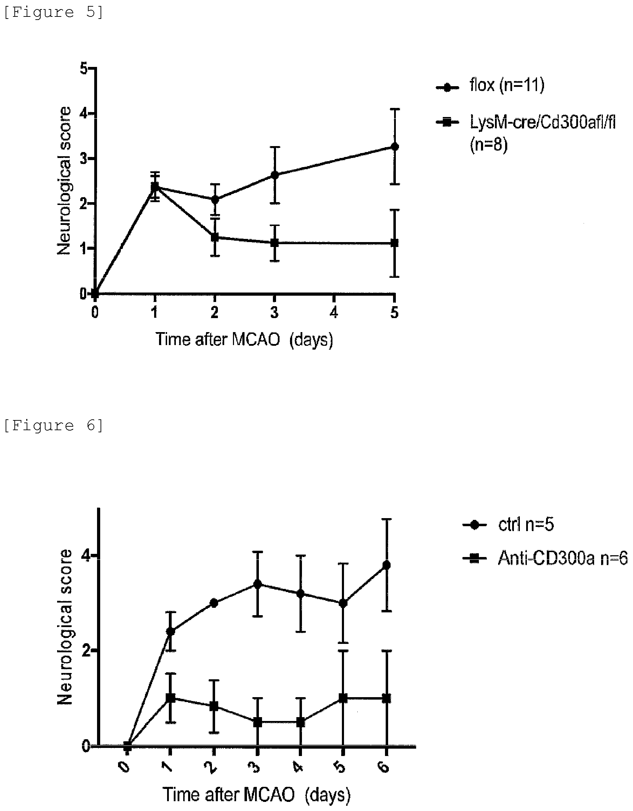

The results are shown in Figure 5. It was found that the neurological damage caused by the cerebral ischemia was milder in the MCAO model mice produced using mice in which CD300a is deficient in macrophages than the MCAO model mice produced using the control mice.

### [Example 2]

Transient cerebral ischemia treatment was performed on wild type mice (C57BL/6J) with the same method as in Reference Example 4. 60 minutes after the treatment, 20 mg/kg of a control antibody (IC17-mouse IgG1 sFc, Chugai Pharmaceutical Co., Ltd.) or an anti-CD300a antibody was intravenously administered. Thereafter, the neurological findings in each mouse were evaluated based on the same scores as in Reference Example 4.

The results are shown in Figure 6. It was found that the neurological damage caused by the transient cerebral ischemia treatment was relatively milder in the group of mice given the anti-CD300a antibody than the group of mice given the control antibody. This suggests that neurological damage caused by cerebral ischemia such as cerebral infarction can be prevented by anti-CD300a antibody.

### [Example 3]

Transient cerebral ischemia treatment was performed on wild type mice (C57BL/6J) with the same method as in Reference Example 4. 3 hours after the treatment, mice having the above-mentioned neurological score of 3 (Circling) were selected, and to each of them 20 mg/kg of control antibody or anti-CD300a antibody was intravenously administered, then the neurological findings were evaluated based on the neurological scores shown in Reference Example 4.

On day 5 after administration of anti-CD300a antibody, the mice were sacrificed, the cerebrum was collected to prepare a cerebral tissue specimen, which was subjected to hematoxylin-eosin staining and observed with an optical microscope (keyence) to measure the area of the infarct region.

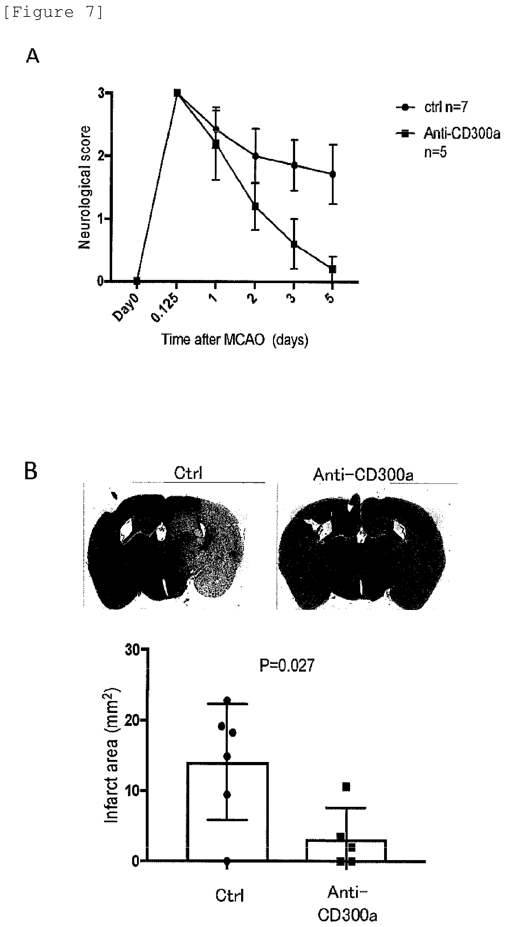

The results are shown in Figure 7. In the group of mice given the anti-CD300a antibody, the neurological score was greatly improved compared to the group of mice given the control antibody, and the staining results also showed that the area of the infarct region was significantly narrower.

This suggests that neurological damage caused by cerebral ischemia such as cerebral infarction can be treated by anti-CD300a antibody.

### [Reference Example 5]

For each of the CD300a^{fl/fl} Lysm-Cre mice and wild type mice (C57BL/6J) produced by the same method as in Reference Example 2, thoracotomy was performed with respiratory assistance after general anesthesia, and the left anterior descending artery was ligated with 6-0 Proline (registered trademark) thread to produce a myocardial infarction model (Mahmoud et al. Nat Prot 2014). One week after the operation, infarction nests were confirmed by Doppler echo, and the mice in which the infarct nest was confirmed were followed up for percent survival.

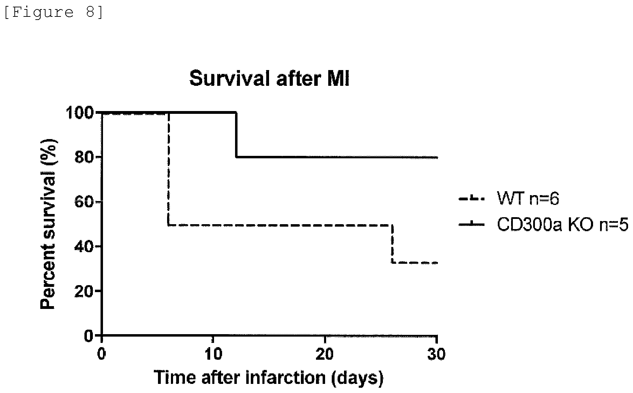

The results are shown in Figure 8. It was found that the survival rate was significantly higher in the myocardial infarction model mice produced using mice in which CD300a is deficient in macrophages than the myocardial infarction model mice produced using the control mice.

## Claims

1. An activity modulator for modulating CD300b-dependent phagocytosis signals in a macrophage, comprising a CD300a-binding substance.

2. The activity modulator according to claim 1, wherein the CD300a-binding substance is a substance that inhibits the activity of CD300a in macrophages.

3. The activity modulator according to claim 1 or 2, wherein the CD300a-binding substance is a substance that inhibits the binding of CD300a to phosphatidylserine.

4. The activity modulator according to any one of claims 1 to 3, wherein the CD300a-binding substance is an antibody or a peptide.

5. The activity modulator according to any one of claims 1 to 3, wherein the CD300a-binding substance is an anti-CD300a antibody.

6. The activity modulator according to claim 5, wherein the anti-CD300a antibody is a CD300a-neutralizing antibody.

7. The activity modulator according to claim 5 or 6, wherein the anti-CD300a antibody is an anti-CD300a monoclonal antibody.

8. A medicine for ischemic diseases comprising the activity modulator according to any one of claims 1 to 7.

9. The medicine for ischemic diseases according to claim 8, wherein the medicine for ischemic diseases is medicine for treating or preventing one or more selected from ischemic heart disease, ischemic brain disease, ischemic bowel disease, ischemic kidney disease, limb ischemia, retinal ischemia, ischemic lung disease and spinal cord ischemia.
